# EUROPÄISCHE PATENTSCHRIFT

(11) **EP 1 464 290 B1**
(45) Veröffentlichungstag und Bekanntmachung des Hinweises auf die Patenterteilung: **11.08.2010**
(21) Anmeldenummer: 04007806.5
(22) Anmeldetag: 31.03.2004
(51) Int. Cl.: A61B 17/28

(54) **Chirurgisches Instrument**
Surgical instrument
Instrument chirurgical

(30) Priorität: 01.04.2003 DE 10314828
(43) Veröffentlichungstag der Anmeldung: 06.10.2004
(73) Patentinhaber: Tuebingen Scientific Medical GmbH, 72074 Tuebingen (DE)
(72) Erfinder: Braun, Marcus, 70563 Stuttgart-Vailhingen (DE)
(74) Vertreter: Winter, Brandl, Fürniss, Hübner, Röss, Kaiser, Polte - Partnerschaft

(56) Entgegenhaltungen:
- DE-A- 10 036 108
- US-A- 5 312 023
- US-A- 5 549 637
- US-A- 5 827 323
- US-B1- 6 309 403

## Beschreibung

Die vorliegende Erfindung betrifft ein chirugisches Instrument für die minimal invasive Chirurgie gemäß dem Oberbegriff des Patentanspruchs 1.

Aus der DE 100 36 108 ist ein chirugisches Instrument dieser Gattung bekannt. Dieses besteht im wesentlichen aus einem Rohrschaft, an dessen einem proximalen Ende ein Instrumentengriff angeordnet ist, mittels dem ein an dem gegenüberliegenden distalen Ende des Rohrschafts angeordneter Instrumentenkopf über Getriebezüge betätigbar ist. Der Instrumentenkopf läßt sich um die Längsachse des Rohrschafts drehen sowie bezüglich des Rohrschafts verschwenken bzw. neigen und trägt zudem einen Effektor in Form einer Art Zange oder Greifer, deren eines Zangenstück schwenkbar am Instrumentenkopf gelagert und mittels des Instrumentengriffs betätigbar ist.

Konkreter ausgedrückt ermöglichen die Getriebezüge, dass zumindest eine erste Bewegung des Instrumentengriffs, beispielsweise auslösbar durch die Handrotation einer Bedienerperson, in eine Rotation des Effektors unter einem bestimmten Übersetzungsverhältnis zu dieser Betätigungsbewegung transformiert wird. Hierdurch ist es möglich, den Effektor trotz der relativ eingeschränkten Bewegungsmöglichkeit einer menschlichen Hand um beispielsweise bis zu 300° zu drehen und damit komplizierte Bewegungsabläufe ohne Umgreifen am Handgriff zu realisieren. Des weiteren wird eine zweite Bewegung des Instrumentengriffs, beispielsweise dessen Abwinkeln bezüglich des Rohrschafts, in eine Neigungsbewegung des Instrumentenkopfs umgesetzt.

Die Getriebezüge innerhalb des Instrumentengriffs und des Rohrschafts sind derart ausgelegt, dass eine weitestgehend entkoppelte Betätigung jeder einzelnen Bewegung des Instrumentenkopfs sowie des Effektors ermöglicht wird. Allderdings sind derartige Getriebe zwangsläufig äußerst kompliziert und benötigen demzufolge auch ausreichend Bauraum. Darüber hinaus ist eine vollständige Entkopplung der einzelnen Bewegungen nicht gänzlich gewährleistet.

Angesichts dieses Stands der Technik ist es eine Aufgabe der vorliegenden Erfindung, ein chirugisches Instrument dieser Gattung zu schaffen, bei welchem Bewegungen eines Instrumentenkopfs voneinander entkoppelt über einen Instrumentengriff ausführbar sind.

Diese Aufgabe wird durch ein chirugisches Instrument mit den Merkmalen gemäß dem Patentanspruch 1 gelöst.

Der Kern der Erfindung besteht demzufolge in der Anordnung eines Bewegungskompensationselements, das in dem Rotations-Getriebezug integriert ist und das bei einer Betätigung des Instrumentengriffs für ein Verschwenken des Instrumentenkopfs gleichzeitig den Rotations-Getriebezug derart antreibt, dass eine durch die Schwenkbewegung des Instrumentenkopfs verursachte Betätigung des Rotations-Getriebezugs kompensiert und damit eine Rotationsbewegung des im Instrumentenkopf gelagerten Effektors verhindert wird. Auf diese Weise ist es möglich, unabhängig von der jeweiligen Abwinkelungsposition des Instrumentenkopfs die Rotationsposition des Effektors einzustellen und/oder aufrecht zu erhalten.

Vorzugsweise besteht der zweite Getriebezug aus dem Bewegungskompensationselement vorzugsweise in Form einer Zahnradwelle, die in der Schwenkachse des Griffstücks am Rohrschaft gelagert ist und die eine Rotations-Betätigung am Griffstück auf eine im Rohrschaft gelagerte Drehwelle überträgt, die in ein Übertragungszahnrad greift, das in einer Schwenkachse des Effektors am Rohrschaft gelagert ist und das mit einem an einer Längsachse des Effektors befestigten Abtriebszahnrad kämmt. Sobald das Griffstück für ein Abwinkeln des Effektors in der Schwenkachse geschwenkt wird, wird die Zahnradwelle entsprechend mit gedreht und treibt über die Drehwelle das Übertragungszahnrad an. Weiter vorzugsweise ist das Übersetzungsverhältnis zwischen der Zahnradwelle und dem Übersetzungszahnrad so bestimmt, dass der Drehwinkel des Übersetzungszahnrads bei einem Verschwenken des Griffstücks jenem Drehwinkel entspricht, den das Abtriebszahnrad bei der Abwinkelung des Effektors ausführt, sodass die Relativposition zwischen Übersetzungszahnrad und Abtriebszahnrad beibehalten wird und eine erzwungene Rotation des Effektors unterbleibt.

Diese Ausgestaltung hat den Vorteil, dass keine zu den ersten und zweiten Getriebezügen zusätzlichen Kompensationseinrichtung vorgesehen werden müssen und daher die gesamte Einrichtung in einem kleinen Bauraum untergebracht werden kann.

Weitere vorteilhafte Ausgestaltungen der Erfindung sind Gegenstand der Unteransprüche.

Die Erfindung wird nachstehend anhand eines bevorzugten Ausführungsbeispiels unter Bezugnahme der begleitenden Zeichnungen näher erläutert.
Fig. 1 zeigt eine Perspektivenansicht eines chirurgischen Instruments gemäß einem bevorzugten Ausführungsbeispiels der Erfindung,
Fig. 2 zeigt einen ersten Getriebezug für ein Verschwenken eines Instrumentenkopfs mittels eines Instrumentengriffs,
Fig. 3 zeigt eine Teilschnittansicht des ersten Getriebezugs im Schwenkbereich des Instrumentenkopfs,
Fig. 4 zeigt einen zweiten Getriebezug für ein Rotieren des Instrumentenkopfs mittels des Instrumentengriffs,
Fig. 5 zeigt eine Teilschnittansicht des zweiten Getriebezugs im Schwenkbereich des Instrumentenkopfs und
Fig. 6a-6c zeigen Schnittdarstellungen eines dritten Getriebezugs im Schwenkbereich des Instrumentenkopfs zur Betätigung einer am Instrumentenkopf gelagerten Zange.

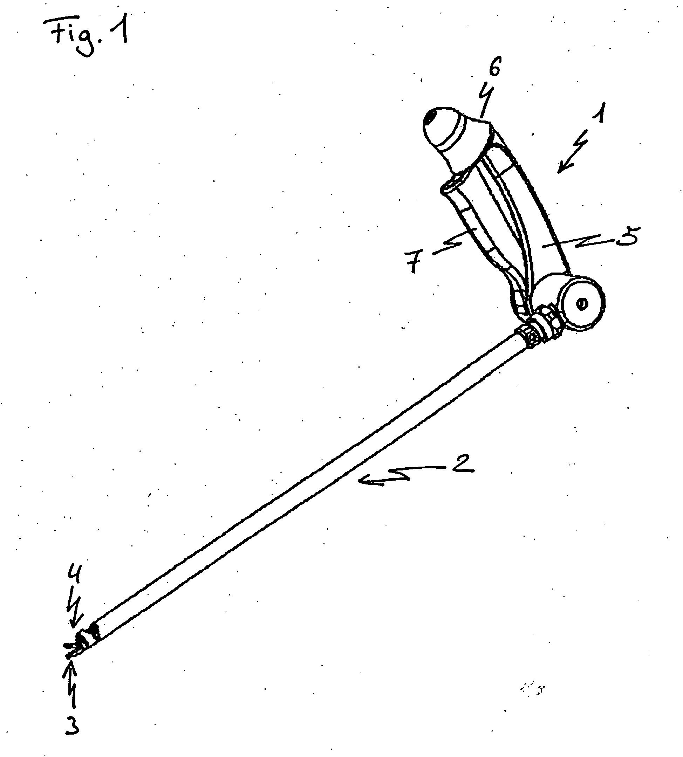

In der Fig. 1 ist ein vollständiges chirurgisches Instrument gemäß einem bevorzugten Ausführungsbeispiel der Erfindung in einer Perspektivenansicht dargestellt. Das erfindungsgemäße chirurgische Instrument hat demzufolge einen multifunktionalen Instrumentengriff 1, der an einem proximalen Ende oder Endabschnitt eines vorzugsweise aus nichtrostendem Stahl, einer Stahllegierung oder einem Kunststoffmaterial gefertigten Rohrschafts 2 angeordnet ist, sowie einem mit einem Effektor 3 bestückten oder bestückbaren Instrumentenkopf 4, der an dem anderen, distalen Ende des Rohrschafts 2 vorgesehen ist.

Generell ist der Instrumentenkopf 4 an dem betreffenden Rohrschaftende so gelagert, dass er bezüglich des Rohrschafts 2 verschwenkt bzw. abgewinkelt werden kann, wohingegen der Effektor 3 in jeder Abwinkelposition des Instrumentenkopfs 4 um dessen Längsachse gedreht bzw. rotiert werden kann, wobei die beiden vorstehend genannten Bewegungen mittels des Instrumentengriffs 1 ausführbar sind. Hierzu sind am Instrumentengriff 1 eine Anzahl von Handhaben oder Betätigungsmechanismen vorgesehen, die über entsprechende Getriebezüge innerhalb des Instrumentengriffs 1 sowie des Rohrschafts 2 mit dem Instrumentenkopf 4 bzw. dem Effektor 3 wirkverbunden sind, um die einzelnen Bewegungen des Instrumentenkopfs 4 und des Effektors 3 unabhängig voneinander, d.h. entkoppelt ausführen zu können.

Konkret besteht der Instrumentengriff 1 aus einem ergomisch geformten Griffstück 5, das schwenk- bzw. neigbar am Rohrschaft 2 montiert ist und an dem eine erste Handhabe 6, vorliegend vorzugsweise in Form eines Drehknopfs sowie eine zweite Handhabe 7, vorliegend vorzugsweise in Form eines Griffhebels gelagert sind. Somit besitzt der Instrumentengriff 1 gemäß dem bevorzugten Ausführungsbeispiel der vorliegenden Erfindung insgesamt drei Betätigungsmechanismen für drei unabhängige Bewegungen des bzw. am Instrumentenkopf 4. An dieser Stelle sei ausdrücklich darauf hingewiesen, dass der Instrumentengriff 1 auch weniger Betätigungsmöglichkeiten aufweisen kann, etwa jeweils nur einen Betätigungsmechanismus für ein Verschwenken des Instrumentenkopfs 4 und Drehen des Effektors 3.

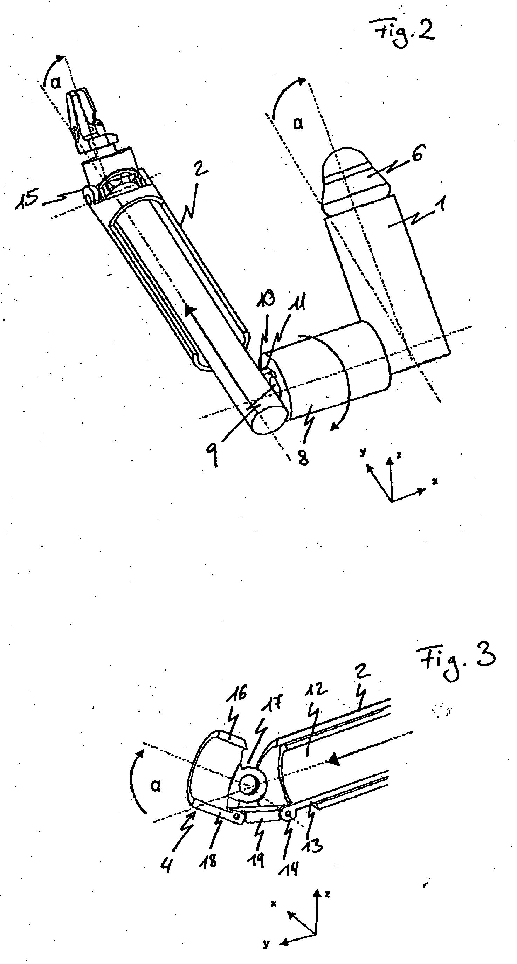

Der äußere Aufbau des Instrumentengriffs 1 insbesondere mit Bezug auf den Betätigungsmechanismus für ein Verschwenken bzw. Abwinkeln des Instrumentenkopfs 4 sowie den zugehörigen Abwinklungs-Getriebezug ist in den Fig. 2 und 3 dargestellt.

Das in Fig. 2 idealisiert dargestellte Griffstück 1 ist über ein fest mit dem Griffstück 1 verbundenes Kulissenelement 8 in Form einer Drehwelle oder Drehscheibe mit dem Rohrschaft 2 schwenkbar verbunden. Hierbei ist die Drehwelle 8 vorzugsweise senkrecht zum Rohrschaft 2 sowie zum Griffstück 1 ausgerichtet und beabstandet das Griffstück 1 vom Rohrschaft 2 derart, dass dieses im wesentlichen parallel zum Rohrschaft 2 an diesem vorbei verschwenkt werden kann.

Die Drehwelle 8, welche einen zentralen Durchgangskanal 9 zur Aufnahme nachfolgend noch beschriebener Getriebeteile ausbildet, ist an ihrer einen, dem Rohrschaft 2 zugewandten Stirnseite mit einer Kulissenführung 10 in Form einer nockenförmigen Nut ausgebildet, in die ein Mitnehmerstift 11 eingreift, welcher an einem im Rohrschaft 2 gelagerten, axial verschiebbaren Schubrohr 12 befestigt ist. Die Nut 10 ist dabei derart ausgebildet, dass bei einer Drehbewegung der Drehwelle 8 durch entsprechendes Schwenken des Griffstücks 1 der Mitnehmerstift 11 in der Nut 10 entlang gleitet und dabei eine Zwangs-Ausgleichsbewegung in Längsrichtung des Rohrschafts 2 ausführt, die auf das Schubrohr 12 übertragen wird und je nach Drehrichtung der Drehwelle 8 zu einer Hin- und Herbewegung des Schubrohrs 12 innerhalb des Rohrschafts 2 führt.

Der dem Kulissenelement 8 abgewandte, distale Endabschnitt des Schubrohrs 12 ist mit einer längs sich ersteckenden Lasche 13 ausgebildet, die über das distale Ende des Schubrohrs 12 vorsteht und an ihrem freien Endabschnitt ein Scharnier bzw. Scharnierösen 14 bildet. Des weiteren ist die Stirnseite des Rohrschafts 2 an seinem distalen Endabschnitt in einem Winkel von vorzugsweise 45° abgeschrägt und mit seitlichen Gelenkösen 15 ausgebildet, an denen der Instrumentenkopf 4 schwenkbar über Gelenkzapfen oder Stifte angelenkt ist. Dieser besteht ebenfalls aus einem Rohrstück 16, dessen eine Stirnseite, an der Anlenkösen 17 für ein Verbinden mit dem Rohrschaft 2 bzw. dessen Gelenkösen 15 ausgebildet sind, ebenfalls in einem Winkel von vorzugsweise 45° abgeschrägt ist und zwar derart, dass sich nach einem Anlenken des Instrumentenkopfs 4 am Rohrschaft 2 die beiden vorstehend genannten Schrägen ergänzen und ein Abwinkeln des Rohrstücks 16 bezüglich des Rohrschafts 2 um ca. 90° vorzugsweise 70° ermöglichen.

Des weiteren ist an der abgeschrägten Stirnseite des Rohrstücks 16 ein Scharnier bzw. Scharnierösen 18 ausgebildet. An den schubrohrseitigen sowie den rohrstückseitigen Scharnierösen 14; 18 ist ein Kipphebel 19 jeweils anscharniert, welcher folglich zur Schwenkachse des Instrumentenkopfs 4 radial nach außen versetzt ist und eine axiale Translationsbewegung des Schubrohrs 12 auf das Rohrstück 16 überträgt, wodurch dieses um dessen Schwenkachse geschwenkt wird.

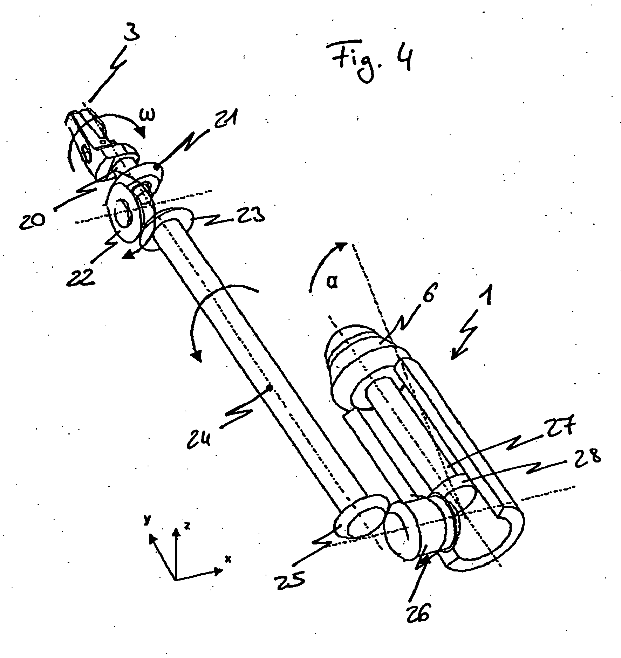

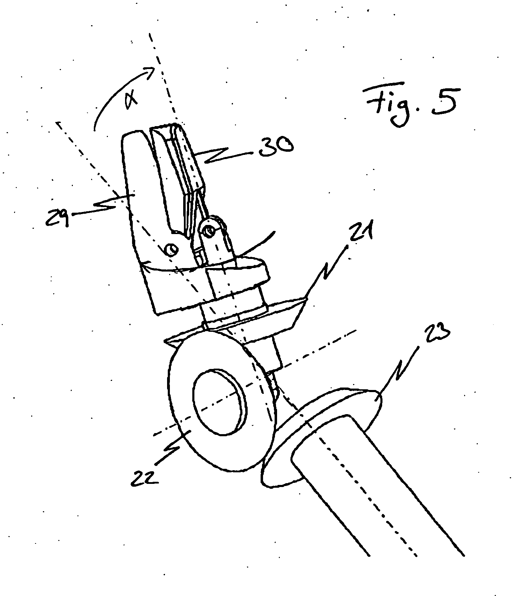

Im nachfolgenden wird anhand der Fig. 4 und 5 der Betätigungsmechanismus für eine Rotation des im Instrumentenkopf 4 gelagerten Effektors 3 sowie der zugehörige Rotations-Getriebezug beschrieben.

Wie noch aus der Fig. 2 zu entnehmen ist, stellt das vorstehend genannte Rohrstück 16 des Instrumentenkopfs 4 gleichzeitig ein Gehäuse bzw. eine Aufnahme für den Effektor 3 dar. Unabhängig davon, um welche Art Effektor es sich dabei handelt, also unabhängig davon, ob als Effektor beispielsweise ein Nadelhalter, ein Greifer, eine Zange oder Schere zum Einsatz kommt, besitzt dieser eine vorzugsweise hohle Rotationsachse 20, die in das Rohrstück 16 des Instrumentenkopfs 4 drehbar eingesetzt und gegen eine Axialbewegung gesichert ist. Die Länge dieser Rotationsachse 20 ist dabei so gewählt, dass diese in etwa im Bereich der Schwenkachse des Instrumentenkopfs 4 endet und an ihrem freien, zu dieser Schwenkachse ragenden Ende mit einem Abtriebs-Stirnrad 21 versehen ist, das drehfest an der Rotationsachse 20 des Effektors 3 befestigt ist. Insbesondere in der Fig. 2 ist die Schwenkachse des Instrumentenkopfs 4 durch eine Strich-Linie durch die Ösen 15 dargestellt.

Wie aus der Fig. 5 ferner zu entnehmen ist, befindet sich auf der Schwenkachse des Instrumentenkopfs 4 ein Drehmoment-Übertragungsstirnrad 22, welches an einem der beiden nicht weiter dargestellten Schwenkzapfen des Instrumentenkopfs 4, welche die idealisiert dargestelle Schwenkachse bilden, drehbar gelagert und mit dem Abtriebs-Stirnrad 21 in Kämmeingriff ist. Das Drehmoment-Übertragungsstirnrad 22 ist wiederum mit einem Antriebs-Stirnrad 23 in Kämmeingriff, welches auf einer innerhalb des Schubrohrs 12 (in den Fig. 4 und 5 nicht dargestellt) drehbar geführten Antriebswelle 24 drehfest montiert ist, wie dies insbesondere in der Fig. 4 gezeigt ist. An einem dem Antriebs-Stirnrad 23 gegenüberliegenden Ende der Antriebswelle 24 ist gemäß der Fig. 4 ein weiteres Drehmoment-Einleitungsstirnrad 25 drehfest angeordnet, welches mit einer Zahnradwelle 26 in Kämmeingriff ist, die in dem innerhalb des Kulissenelements 8 ausgebildeten zentralen Durchgangskanal 9 gelagert ist.

Das Kulissenelement 8 ist in der Fig. 4 nicht weiter dargestellt.

Die Zahnradwelle 26 ist schließlich mit einer Betätigungswelle 27, bzw. einem daran befestigten Stirnrad 28 innerhalb des Griffstücks 1 in Kämmeingriff, welche mit der einen Handhabe, vorliegend dem Drehknopf 6, fest verbunden ist.

Bei einer Betätigung des Drehknopfs 6 wird dessen Drehbewegung über die Betätigungswelle 27 innerhalb des Griffstücks 1, die Zahnradwelle 26, die daran sich anschließende Antriebswelle 24 innerhalb des Schubrohrs 12 sowie das Übertragungsstirnrad 22 auf den Effektor 3 übertragen und dieser gedreht. Der Drehknopf 6 wird dabei in vorteilhafter Weise mit den Fingern insbesondere mit Daumen und Zeigefinder der Bedienerhand betätigt, während das Griffstück 1 in Hand gehalten wird. Es ist somit möglich, jede beliebige Rotation am Effektor 3 zu erzeugen, ohne dass der Bediener am Griffstück 1 selbst umgreifen muss. An dieser Stelle sei ferner noch darauf hingewiesen, dass die Antriebswelle 24 sowie das Schubrohr 12 in Axialrichtung relativ beweglich zueinander angeordnet sind. D.h. eine durch ein Verschwenken des Handgriffs 1 ausgelöste Drehung des Kulissenelements 8 bewirkt zwar eine Translationsbewegung des Schubrohrs 12. Gleichzeitig wird jedoch die Antriebswelle 24 in Position, d.h. in Kämmeingriff mit der Zahnradwelle 26 gehalten, wodurch das Schubrohr 12 eine axiale Relativbewegung zum Rohrschaft 2 wie auch zur Antriebswelle 24 ausführt.

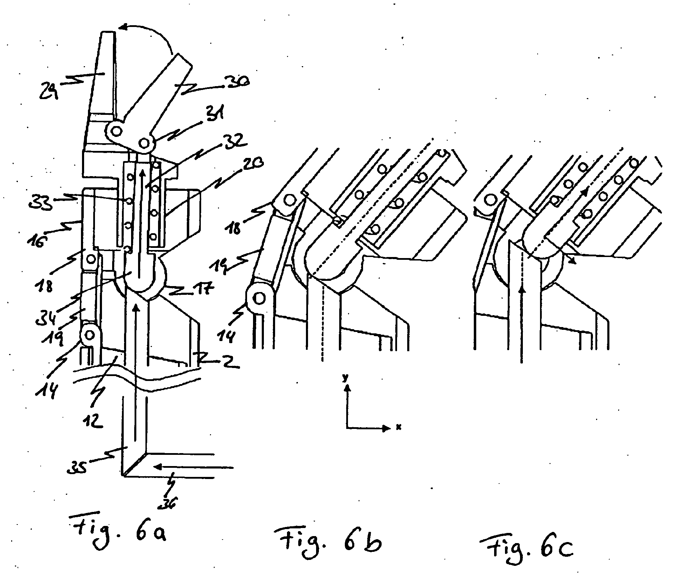

Schließlich wird nachfolgend der Betätigungsmechanismus für den Effektor 3, d.h. dessen Funktionen selbst sowie den zugehörigen Effektor-Getriebezug anhand der Fig. 5 und 6a-6c beschrieben.

Gemäß der Fig. 5 ist der Effektor 3 in dem vorliegenden Ausführungsbeispiel der Erfindung als eine Zange mit einer feststehenden sowie einer bewegbaren, d.h. schwenkbaren Zangenbacke 29; 30 ausgebildet. Die feststehende Zangenbacke 29 bildet zusammen mit der Rotationswelle 20 des Effektors 3 eine Einheit und ist vorzugsweise einstückig mit der Rotationswelle 20 ausgebildet, wohingegen die bewegbare Zangenbacke 30 an einem Ende schwenkbar an der feststehenden Zangenbacke 29 angelenkt ist.

Die bewegbare Zangenbacke 30 bildet eine Anlenkstelle 31 für einen Schubbolzen 32, der innerhalb der Rotationswelle 20 relativ verschiebbar gelagert ist, sodass durch dessen Axialverschiebung eine Schwenkbewegung der bewegbaren Zangenbacke 30 mit möglichst großer Übersetzung bewirkt wird. Der Schubbolzen 32 ist, wie dies insbesondere in den Fig. 6a - 6c dargestellt wird, mittels einer Feder 33 axial in Öffnungsrichtung der Zange vorgespannt, die innerhalb der Rotationsachse 20 den Schubbolzen 32 umgibt. Hierfür bildet der Schubbolzen 32 einen Wellenabsatz, an dem sich die Vorspannfeder 33 an ihrem einen Ende abstützt. Das andere Ende der Vorspannfeder 33 ist gegen die feststehende Zangenbacke 29 abgestützt. Ein aus der Rotationsachse 20 in Richtung zur Schwenkachse des Instrumentenkopfs 4 herausragendes Endstück 34 des Schubbolzens 32 ist kugelkopfförmig ausgebildet, wobei der Radius des Kugelkopfs 34 vorliegend vorzugsweise ca. 2.5 mm beträgt.

Die vorstehend genannte Antriebswelle 24 für das rotorische Antreiben des im Instrumentenkopf 3 gelagerten Effektors 3 ist mit einer im wesentlichen durchgehenden Axialbohrung (nicht weiter gezeigt) versehen. In dieser Axialbohrung ist ein Schubstab 35 axialverschieblich, sowie relativ zur Antriebswelle 24 drehbar geführt, dessen dem Schubbolzen 32 zugewandte Stirnseite entsprechend den Abschrägungen der rohrschaftseitigen wie auch schubrohrseiten distalen Stirnseiten d.h. vorzugsweise 45° in die selbe Richtung abgeschrägt ist. Der Schubbolzen 32 ist mittels der Feder 33 gegen diese abgeschrägte Stirnfläche des Schubstabs 35 vorgespannt und kommt mit dieser in Anlage. Dabei ist die Anlagefläche zwischen dem Schubstab 35 und dem Schubbolzen 32 aufgrund der vorstehend beschriebenden Kugelkopfform des Bolzens 32 im wesentlichen punktförmig und zwar unabhängig von dem Abwinkelungsgrad des Instrumentenkopfs 4 sowie unabhängig von der Rotationsposition des Effektors 3.

Wie aus der Fig. 6a zu erkennen ist, sind der Schubbolzen 32 wie auch der Schubstab 35 für den Fall, dass die Abwinkelung des Instrumentenkopfs 4 bezüglich des Rohrschafts 2 im wesentlichen 0° beträgt, axial zueinander ausgerichtet. Darüber hinaus ist der Schubbolzen 32 in dieser Stellung des Instrumentenkopfs 4 so positioniert, dass der Mittelpunkt des Kugelkopfs 34 des Schubbolzens 32 in etwa in der Schwenkachse des Instrumentenkopfs 4 liegt.

Der Schubstab 35 ist an seinem proximalen Ende über ein nicht im Einzelnen gezeigtes Getriebe 36 mit dem Betätigungshebel 7 verbunden, der, wie Eingangs dieser Beschreibung bereits kurz ausgeführt wurde, schwenkbar am Griffstück 1 gelagert ist.

Die Funktionsweise des erfindungsgemäßen chirurgischen Instruments wird nachfolgend im Einzelnen beschrieben.

Eine Rotation des im Instrumentenkopf 4 gelagerten Effektors 3 erfolgt durch eine Betätigung des an einem Ende des Griffstücks 1 gelagerten Drehkopfs 6, wobei der Drehknopf 6 wie vorstehend bereits ausgeführt wurde, soweit um seine Drehachse gedreht werden kann, dass eine ca. 360° Rotation am Effektor 3 realisiert wird, ohne dass am Griffstück 1 notwendiger Weise umgegriffen werden muss. Diese Drehbewegung wird über die Betätigungswelle 27 auf die Zahnradwelle 26 übertragen, welche wiederum ihre Drehbewegung auf die innerhalb des Schubrohrs 12 verlaufende Antriebswelle 24 weiter gibt. Die Drehbewegung der Antriebswelle 24 bewirkt eine Drehbewegung des Übertragungsstirnrads 22, welches die Schwenkachse des Instrumentenkopfs 4 quasi überbrückt und damit eine Rotationsbewegung des Effektors 3 innerhalb des Rohrstücks 16 des Instrumentenkopfs 4 um die Rohrstückachse auslöst.

Um eine Abwinkelung, d.h. eine Schwenkbewegung des Instrumentenkopfs 4 und damit des Effektors 3 zu bewirken, muß gemäß dem vorliegenden Ausführungsbeispiel das gesamte Griffstück 1 um die Längsachse des Kulissenelements 8 geschwenkt werden. In anderen Worten ausgedrückt, bewirkt eine Schwenkbewegung des Griffstücks 1 bezüglich des Rohrschafts 2 eine Drehbewegung des drehfest mit dem Griffstück 1 verbundenen Kulissenelements 8. Gleichzeitig jedoch wird aufgrund der Tatsache, dass durch den Kämmeingriff zwischen der Betätigungsachse 27 und der Zahnradwelle 26 eine Art Selbsthemmung durch Reibung (Wirkungsgrad des Getriebes) entsteht, welche ggf. durch leichtes Halten des Betätigungsknopfs 6 sowie durch die Haftreibung zwischen Betätigungsknopf 6 und Griffstück 1 noch unterstützt wird, die Zahnradwelle 26 mit dem Kulissenelement 8 mit gedreht.

Die Drehung des Kulissenelements 8 wird über die Kulisse bzw. Nut 10 an der Stirnseite des Elements 8 sowie den Mitnehmerzapfen 11 in eine Axialbewegung des Schubrohrs 12 übertragen, die über den anscharnierten Kipphebel 19 zu einer Schwenkbewegung des Instrumentenkopfs 4 um dessen Schwenkachse transformiert wird. Diese Schwenkbewegung führt jedoch zwangsläufig auch das Abtriebsstirnrad 21 aus, welches an der Rotationsachse des Effektors 3 fixiert und mit dem Übertragungsstirnrad 22 in Kämmeingriff ist. Würde demzufolge das Übertragungsstirnrad 22 bei dieser Betätigungsart, d.h. der Verschwenkbetätigung, feststehen, würde die Schwenkbewegung des Instrumentenkopfs 3 ein gleichläufiges Abrollen des Abtriebsstirnrads 21 an dem Übertragungsstirnrad 22 bewirken und somit zwangsläufig zu einer überlagerten Rotationsbewegung des Effektors 3 führen.

Wie vorstehend jedoch ausgeführt wurde, wird die Zahnradwelle 26 bei einer Schwenkbewegung des Griffstücks 1 zusammen mit dem Kulissenelement 8 mitgedreht und treibt dabei die Antriebswelle 24 innerhalb des Schubrohrs 12 an. Die Übersetzung zwischen der Zahnradwelle 26 und der Antriebswelle 24 ist dabei so berechnet, dass das Übertragungszahnrad 22 durch die Anriebswelle 24 um einen solchen Drehwinkel gedreht wird, der dem Drehwinkel entspricht, welcher durch das Abtriebszahnrad 21 bei einer entsprechenden Abwinkelung des Instrumentenkopfs 4 hervorgerufen wird, wodurch sich beide Drehungen aufgrund ihrer Gegenläufigkeit kompensieren. Bei dieser Konstellation wird die Relativposition zwischen dem Übertragungsstirnrad 22 und dem Abtriebsstirnrad 21 auch während der Abwinkelungsbewegung des Instrumentenkopfs 4 beibehalten, so dass der Effektor 3 in jeder Abwinkelungsposition des Instrumentenkopfs 4 bzw. während einer Abwinkelungsbewegung in seiner augenblicklichen Rotationsposition bezüglich des Instrumentenkopfs 4 gehalten wird.

Um eine Betätigung des Effektors 3 d.h. dessen Funktion selbst zu bewirken, ist in dem vorliegenden bevorzugten Ausführungsbeispiel der am Griffstück 1 schwenkbar gelagerte Hebel 7 vorgesehen. Wie bereits vorstehend zu den Fig. 6a-6c ausgeführt wurde, ist der Hebel 7 über ein nicht weiter dargestelltes Umlenkgetriebe oder einen entsprechenden Gelenkmechanismus mit dem in der Drehwelle 24 gelagerten Schubstab 35 wirkverbunden, welcher sich bei einer entsprechenden Betätigung des Hebels 7 relativ zur Drehwelle 24 axial hin- und herbewegt. Auch ein einfacher Bowdenzug oder ein Umlenkhebel wäre für eine Kraftübertragung auf den Schubstab 35 denkbar.

Die Fig. 6a zeigt nunmehr die Relativlage des Schubstabs 35 sowie des Schubbolzens 32 in 0°-Abwinkelposition des Instrumentenkopfs 4 bei geöffneter Zange, die Fig. 6b zeigt die Relativlage des Schubstabs 35 sowie des Schubbolzens 32 in ca. 45°-Abwinkelposition des Instrumentenkopfs 4 bei geöffneter Zange und die Fig. 6c zeigt die Relativlage des Schubstabs 35 sowie des Schubbolzens 32 in ca. 45°-Abwinkelposition des Instrumentenkopfs 4 bei geschlossener Zange.

Wie aus den Fig. 6a-6c zu entnehmen ist, wird der Schubbolzen 32 durch die Vorspannkraft der Feder 33 in ständiger Anlage mit der abgeschrägten oder gefasten distalen Stirnfläche des Schubstabs 35 gehalten. Bei einer Verschiebung des Schubstabs 35 in Richtung Instrumentenkopf 4 im Fall einer 0°-Abwinkelung des Instrumentenkopfs 4 gemäß der Fig. 6a wird der Schubbolzen 32 mit gleicher Geschwindigkeit und Wegstrecke wie der Schubstab 35, d.h. ohne Übersetzung, entgegen der Vorspannkraft der Feder 33 verschoben, wodurch die daran angelenkte Zangenbacke 30 in Schließrichtung verschwenkt wird.

An dieser Stelle sei darauf hingewiesen, dass durch die Verschiebetätigkeit des Schubstabs 35 der Schubbolzen 32, d.h. insbesondere der Mittelpunkt des Bolzenkopfradius nur annähernd auf der Schwenkachse des Instrumentenkopfs 4 verbleibt, sich also bei einer Abwinkelungsbewegung des Instrumentenkopfs 4 auf einer Art Kreisbahn bewegt. Wie jedoch eingangs der Figurenbeschreibung bereits ausgeführt wurde, sind die Stellwege für ein Öffnen und Schließen beispielsweise der Zange aufgrund der eingestellten Übersetzungen so gering, dass der Kreisbahnradius zwar theoretisch berechenbar ist, jedoch bereits schon aus fertigungstechnischen Gründen (Eigenelastizität der verwendeten Materialien, Maßtoleranzen und Spiel an den Gelenkverbindungen und Getriebeteilen) keinen relevanten Einfuß auf Zangenstellung hat. In anderen Worten ausgedrückt, wird die Zangenstellung durch die Position des Hebels 7 bestimmt, welcher wiederum von einer Bedinerperson gehalten wird und damit beispielsweise auch unkontrollierbaren Handbewegungen (Zitterbewegungen) unterliegt. Derartige aufgrund manueller Betätigungen erzeugte Störungen sind im Vergleich zu jenen Störungen, welche durch die vorstehend beschriebene Kreisbahnbewegung erzeugt werden, wesentlich größer und daher praktisch alleinig maßgeblich.

D.h. unabhängig von der aktuellen Position des Schubstabs 35 bzw. des Schubbolzens 32 bewirkt eine Abwinkelung des Instrumentenkopfs 4 generell nicht nur ein Verschwenken des Schubbolzens 32 bezüglich des Schubstabs 35 sondern auch ein geringes Abgleiten des Bolzenkopfs 34 auf der abgeschrägten Stirnfäche des Schubstabs 35. Durch diese geringe Abgleitbewegung bleibt der Anlagekontakt des Schubbolzens 32 auf der Stirnfläche erhalten, wobei jedoch nur eine solche Ausgleichs-Längsbewegung des Schubbolzens 32 in Folge seiner Abgleitbewegung erfolgt, die zu keiner praktisch relevanten Veränderung der Schließ- oder Öffnungsposition am Effektor 3 führt. Gleichzeitig jedoch wird eine Art Kraftumlenkmechanismus geschaffen, um eine Längsbewegung des Schubstabs 35 in eine Längsbewegung des nunmehr im Winkel zum Schubstab 35 stehenden Schubbolzens 32 durch die Abschrägung der Schubstab-Stirnfläche zu bewirken.

In anderen Worten ausgedrückt, wird der Schubstab 35 bei einer Abwinkelungsposition > 0° gemäß der Fig. 6b in Schließrichtung des Effektors 3 verschoben, wie dies in der Fig. 6c dargestellt ist, gleitet die abgeschrägte Stirnfläche des Schubstabs 35 längs am Bolzenkopf 34 vorbei und übt dabei eine Vorschubkraft auf den Schubbolzen 32 aus, der sich dementsprechend in Schließrichtung des Effektors 3 bewegt.

Zusammenfassend läßt sich ein Erfindungsgedanke also beschreiben als ein chirugisches Instrument mit einem Rohrschaft 2, an dessen proximalem Ende ein einen Effektor 3 drehbar lagernden Instrumentenkopf 4 schwenkbar gelagert ist und an dessen distalem Ende ein Instrumentengriff 1 angeordnet ist, der über einen Abwinkelungs-Getriebezug eine Schwenk- bzw. Abwinkelbewegung des Instrumentenkopfs 4 und über einen Rotations-Getriebezug eine Rotationsbewegung des Effektors 3 bewirkt. Ein Bewegungskompensationselement vorzugsweise in Form einer Zahnradwelle 26 ist in dem Rotations-Getriebezug integriert, das bei einer Betätigung des Abwinklungs-Getriebezugs über den Instrumentengriff 1 mit betätigt wird und den Rotations-Getriebezug derart antreibt, dass eine durch die Schwenkbewegung des Instrumentenkopfs 4 verursachte Betätigung des Rotations-Getriebezugs bzw. Teile davon kompensiert wird.

## Patentansprüche

1. Chirurgisches Instrument bestehend aus einem Rohrschaft (2), an dessen einem Ende ein Effektor (3) drehbar sowie schwenkbar gelagert ist und an dessen anderem Ende ein Instrumentengriff (1) angeordnet ist, der über einen ersten Getriebezug eine Schwenkbewegung und über einen zweiten Getriebezug eine Rotationsbewegung des Effektors (3) bewirkt, und mit einem Bewegungskompensationselement (26), das in
dem zweiten Getriebezug integriert ist und das bei einem Antrieb des ersten Getriebezugs mittels des Instrumentengriffs (1) für ein Verschwenken des Effektors (3) den zweiten Getriebezug derart antreibt,
dass ein durch die Schwenkbewegung des Effektors (3) verursachter Antrieb des zweiten Getriebezugs kompensiert wird, wobei der Instrumentengriff (1) einen mit dem ersten Getriebezug wirkverbundenen Verschwenkungs-Betätigungsmechanismus (8, 9, 10, 11) und einen mit dem zweiten Getriebezug wirkverbundenen Rotations-Betätigungsmechanismus (6, 26, 27) hat, die unabhängig voneinander betätigbar sind, um den in einem Instrumentenkopf (4) gelagerten Effektor (3) zu drehen und den Instrumentenkopf (4) mit samt dem Effektor (3) abzuwinkeln, **dadurch gekennzeichnet, dass**
der Verschwenkungs-Betätigungsmechanismus (8, 9, 10, 11) eine Schwenklagerung (8) des Instrumentengriffs (1) am Rohrschaft (2) beinhaltet, in der das Bewegungskompensationselement (26) vorzugsweise in Form einer Zahnradwelle so angeordnet ist, dass dieses durch eine Schwenkbewegung des Instrumentengriffs (1) mit drehbar ist, um den zweiten Getriebezug anzutreiben, wobei
der zweite Getriebezug folgende Elemente hat:
das Bewegungskompensationselement (26), welches auch als Bestandteil des Rotations-Betätigungsmechanismus (6, 27, 26) des Griffstücks (1) eine Rotations-Betätigung auf eine im Rohrschaft (2) gelagerte Drehwelle (24) überträgt, die in ein Übertragungszahnrad (22) greift, das in einer Schwenkachse des Effektors (3) am Rohrschaft (2) gelagert ist und das mit einem an einer Längsachse des Effektors (3) befestigten Abtriebszahnrad (21) kämmt.

2. Chirurgisches Instrument nach Anspruch 1, **dadurch gekennzeichnet, dass**
das Bewegungskompensationselement (26) mittels des Instrumentengriffs (1) insbesondere mittels des Rotations-Betätigungsmechanismus (6, 27, 26) als auch mittels des Verschwenkungs-Betätigungsmechanismus (8, 9, 10, 11) gleichzeitig antreibbar ist.

3. Chirurgisches Instrument nach Anspruch 1, **dadurch gekennzeichnet, dass**
der erste Getriebezug folgende Elemente hat:
ein die Schwenklagerung des Griffstücks (1) bildendes, sowie mit dem Griffstück (1) drehfest verbundenes Kulissenelement (8), das mit einem im Rohrschaft (2) gelagerten Schubrohr (12) so in Eingriff ist, um eine Schwenkbewegung des Schubrohrs (12) zu transformieren, welches an seinem dem Instrumentenkopf (4) zugewandten Ende über einen Gelenkmechanismus (13, 14, 18) mit dem Instrumentenkopf (4) wirkverbunden ist um diesen bei einer Axialverschiebung um seine Schwenkachse mit dem Rohrschaft (2) zu verschwenken.

4. Chirurgisches Instrument nach Anspruch 3, **dadurch gekennzeichnet, dass** das Kulissenelement (8) zu einer Drehwelle geformt ist, an deren einer Stirnseite eine aus einer nutenförmigen Nockebbahn bestehenden Kulisse (10) ausgebildet ist in die ein am Schubrohr (12) befestigter Mitnehmerstift (11) eingreift.

5. Chirurgisches Instrument nach nach Anspruch 3, **dadurch gekennzeichnet, dass** der Rotations-Betätigungsmechanismus (6, 27, 26) aus einer am Instrumentengriff (1) gelagerten Handhabe vorzugsweise in Form eines Drehknopfs (6) besteht, die mit einer im Griff (1) gelagerten Betätigungswelle (27) verbunden ist, welche mit der Zahnradwelle (26) über ein Zahnradgetriebe (28) in Wirkeingriff ist.

6. Chirurgisches Instrument nach Anspruch 5, **dadurch gekennzeichnet, dass** das Übersetzungsverhältnis des Zahnradgetriebes (28) so bestimmt ist, dass bei einer Verschwenkung des Instrumentengriffs (1) um einen beliebigen Winkel ( alpha ), welches eine Abwinkelung des Instrumentenkopfs (4) um einen Winkel ( beta ) bewirkt, die Drehwelle (24) um einen solchen Winkel gedreht wird, dass das auf der Schwenkachse es Instrumentenkopfs (4) gelagerte Übertragungszahnrad (22) um den gleichen Winkel ( beta) wie der Instrumentenkopf (4) gedreht wird, wodurch die Relativposition zwischen Übertragungszahnrad (22) und Abtriebszahnrad (21) unverändert bleibt.

## Claims

1. A surgical instrument comprising a tube shaft (2), at one end of which an effector (3) is rotably and pivotably supported and at the other end of which an instrument handle (1) is provided, which effects via a first gear train a pivoting movement and via a second gear train a rotating movement of said effector (3) an d said instrument comprises a movement compensation element (26), which is integrated into the second gear train and which, in case the first rear train is driven via said instrument handle (1) for pivoting said effector (3), drives the second gear train such that a movement of said second gear train resulting from the pivoting movement of the effector (3) will be compensated, wherein said instrument handle (1) comprises a pivoting activation mechanism (8, 9, 10, 11) operatingly connected with said first gear train and a rotating activation mechanism (6, 26, 27) operatingly connected with the second gear train which mechanisms are operatable independendly from each other for rotating said effector (3) supported inside an instrument head (4) and for pivoting the instrument head (4) together with the effector (3), **characterized in that** Said pivoting activation mechanism (8, 9, 10, 11) comprises a pivotation support (8) of said instrument handle (1) at said tube shaft (2), in which support (8) said movement compensation element (26) especially in the form of a gear shaft is provided such that said element (26) rotates together with a pivoting movement of said instrument handle (1) for driving said second gear train wherein said second gear train comprises the following elements:
Said movement compensation element (26) which as a member of said rotating activation mechanism (6, 27, 26) of said handle (1) transfers a rotating operation to a rotating shaft (24) supported within said tube shaft (2) which rotating shaft (24) is in tooth connection with a transfer tooth gear (22) which is supported at said tube shaft (2) within a pivoting axis of said effector (3) and which is in tooth connection with a driven tooth gear (21) fixed at an longitudinal axle of said effector (3).

2. A surgical instrument according to claim 1, **characterized in that** said movement compensation element (26) is coincidentally drivable by said instrument handle (1) especially said rotating activation mechanism (6, 27, 26) as well as by said pivoting activation mechanism (8, 9, 10, 11).

3. A surgical instrument according to claim 1, **characterized in that** said first gear train comprises the following elements:
a crank element (8) forming said pivoting support for said handle (1) and being fixedly connected with said handle (1), which crank element (8) is in operation connection with a push tube (12) supported inside said tube shaft in order to transform a pivoting movement of said push tube (12), which at its one end facing said instrument head (4) is operatively connected with said instrument head (4) via a link mechanism (13, 14, 18), for pivoting the instrument head (4) arround its pivoting axis with said tube shaft (2) in case of an axial shift of said push tube (12).

4. A surgical instrument according to claim 3, **characterized in that** said crank element (8) is formed as a rotating shaft, at one axial end side of which a crank (10) is formed by a groove-like cam path in which an actuator pin (11) is engaged.

5. A surgical instrument according to claim 3, **characterized in that** said rotating activation mechanism (6, 27, 26) comprises a handle especially in the form of a rotating knob (6) supported at said instrument handle (1), which knob (6) is connected with an operation shaft (27) supported inside said instrument handle (1) which shaft (27) is in operating engagement with said gear shaft (26) via a spur gear (28).

6. A surgical instrument according to claim 5, **characterized in that** said transmission ration of said spur gear (28) is defined such that in case of a pivotation of said instrument handle (1) by an arbitrary angle (α) which results in a pivotation of said instrument head (4) by an angle (β) said rotating shaft (24) is rotated by an angle, such that said transmission gear (22) supported on the pivoting axis of said instrument head (4) is rotated by the same angle (β) as the instrument head (4) whereby the relative position between the transmission gear (22) and the driven gear (21) is maintained unchanged.

## Revendications

1. Instrument chirurgical composé d'une tige tubulaire (2) à l'une des extrémités de laquelle est logé un effecteur (3) pouvant être pivoté et articulé et à l'autre extrémité de laquelle est disposée une poignée d' instrument (1) qui induit via un premier train d'engrenages un mouvement pivotant et via un deuxième train d'engrenages un mouvement de rotation de l'effecteur (3) et avec un élément de compensation de mouvement (26) qui est intégré dans le deuxième train d'engrenages et qui entraîne le deuxième train d'engrenages en cas d'actionnement du premier train d'engrenages à l'aide de la poignée d'instrument (1) pour un pivotement de l'effecteur (3) de telle manière qu'un actionnement du deuxième train d'engrenages provoqué par le mouvement de pivotement de l'effecteur (3) est compensée, la poignée d'instrument (1) ayant un mécanisme d'actionnement de pivotement (8, 9, 10, 11) relié de manière active avec le premier train d'engrenages et un mécanisme d'actionnement de rotation (6, 26, 27) relié de manière active avec le deuxième train d'engrenages qui peuvent être actionnés indépendamment l'un de l'autre, pour pivoter l'effecteur (3) logé dans une tête d'instrument (4) et pour plier la tête d'instrument (4) avec tout l'effecteur (3), **caractérisé en ce que** le mécanisme d'actionnement de pivotement (8, 9, 10, 11) contient un logement pivotant (8) de la poignée d'instrument (1) sur la tige tubulaire (2), dans lequel l'élément de compensation de mouvement (26) est disposé de préférence sous la forme d'un arbre formant pignon de telle sorte que cet élément peut être pivoté par un mouvement pivotant de la poignée d'instrument (1), pour actionner le deuxième train d'engrenages,
le deuxième train d'engrenages ayant les éléments suivants :
l'élément de compensation de mouvement (26), qui communique également en tant que mécanisme d'actionnement de rotation (6, 27, 26) de la poignée (1) un actionnement de rotation sur un arbre rotatif (24) logé dans la tige tubulaire (2) qui vient en prise dans un pignon de transmission (22) qui est logé dans un axe rotatif de l'effecteur (3) sur la tige tubulaire (2) et qui s'engrène avec un pignon de sortie (21) fixé sur un axe longitudinal de l'effecteur (3).

2. Instrument chirurgical selon la revendication 1, **caractérisé en ce que**
l'élément de compensation de mouvement (26) peut être actionné simultanément au moyen de la poignée d'instrument (1) en particulier au moyen du mécanisme d'entraînement de rotation (6, 27, 26) ainsi qu'au moyen du mécanisme d'actionnement de pivotement (8, 9, 10, 11).

3. Instrument chirurgical selon la revendication 1, **caractérisé en ce que**
le premier train d'engrenages a les éléments suivants :
un élément coulissant (8) formant le logement pivotant de la poignée (1), et relié de manière résistante à la torsion à la poignée (1) qui est ainsi en prise avec un tube télescopique (12) logé dans la tige tubulaire (2) pour transformer un mouvement de pivotement du tube télescopique (12) qui est relié de manière active au niveau de son extrémité tournée vers la tête d'instrument (4) via un mécanisme d'articulation (13, 14, 18) avec la tête d'instrument (4) afin de pivoter celle-ci en cas de déplacement axial autour de son axe pivotant avec la tige tubulaire (2).

4. Instrument chirurgical selon la revendication 3, **caractérisé en ce que** l'élément coulissant (8) est façonné en arbre rotatif, au niveau du côté frontal duquel est formée une coulisse (10) se composant d'une glissière-came en forme de rainure dans laquelle une broche d'entraînement (11) fixée sur le tube télescopique (12) vient en prise.

5. Instrument chirurgical selon la revendication 3, **caractérisé en ce que** le mécanisme d'actionnement de rotation (6, 27, 26) se compose d'une manette logée sur la poignée d'instrument (1) de préférence sous la forme de bouton rotatif (6) qui est reliée à un arbre d'actionnement (27) logé dans la poignée (1) qui est en prise active avec l'arbre formant pignon (26) via un engrenage (28).

6. Instrument chirurgical selon la revendication 5, **caractérisé en ce que** le rapport de transformation de l'engrenage (28) est déterminé de telle sorte que, en cas de pivotement de la poignée d'instrument (1) autour d'un angle arbitraire (alpha) qui induit un pliage de la tête d'instrument (4) autour d'un angle (bêta), l'arbre rotatif (24) est pivoté autour d'un angle tel que le pignon de transmission (22) logé sur l'axe pivotant de la tête d'instrument (4) est pivoté autour du même angle (bêta) que la tête d'instrument (4), moyennant quoi la position relative entre le pignon de transmission (22) et le pignon de sortie (21) reste inchangée.
